# EUROPEAN PATENT SPECIFICATION

(11) **EP 1 075 525 B1**
(45) Date of publication and mention of the grant of the patent: **06.10.2004**
(21) Application number: 00909319.6
(22) Date of filing: 04.03.2000
(51) Int. Cl.: C12N 15/54, C12N 9/12, C12Q 1/68

(54) **DNA POLYMERASE FROM PYROBACULUM ISLANDICUM**
DNA POLYMERASE AUS I(PYROBACULUM ISLANDICUM)
Polymerase-ADN de Pyrobaculum Islandicum

(30) Priority: 06.03.1999 EP 99104539; 02.09.1999 EP 99117245
(43) Date of publication of application: 14.02.2001
(73) Proprietor: Roche Diagnostics GmbH, 68298 Mannheim (DE)
(72) Inventor: KAEHLER, Markus, D-25524 Itzehoe (DE); FREY, Bruno, D-82377 Penzberg (DE); SOBEK, Harald, D-82377 Penzberg (DE); ANTRANIKIAN, Garabed, D-21218 Seevetal-Hittfeld (DE)
(86) International application number: PCT/EP2000/001917
(87) International publication number: WO 2000/053772

(56) References cited:
- EP-A- 0 624 641
- WO-A-93/25691
- WO-A-99/07837
- BURGGRAF S ET AL: "An intron within the 16S ribosomal RNA gene of the archaeon Pyrobaculum aerophilum." PROCEEDINGS OF THE NATIONAL ACADEMY OF SCIENCES OF THE UNITED STATES OF AMERICA 1993, vol. 90, no. 6, 1993, pages 2547-2550, XP002139882 ISSN: 0027-8424
- M. MOLITOR ET AL.: "A dissimilatory sirohaem-sulfite-reductase-type protein from the hyperthermophilic archaeon Pyrobaculum islandicum" MICROBIOLOGY, vol. 144, 1998, pages 529-541, XP002139883 READING, UK
- EDGELL DAVID R ET AL: "Gene duplications in evolution of archaeal family B DNA polymerases." JOURNAL OF BACTERIOLOGY 1997, vol. 179, no. 8, 1997, pages 2632-2640, XP002139884 ISSN: 0021-9193 cited in the application
- SCHLEPER CHRISTA ET AL: "Characterization of a DNA polymerase from the uncultivated psychrophilic archaeon Cenarchaeum symbiosum." JOURNAL OF BACTERIOLOGY DEC., 1997, vol. 179, no. 24, December 1997 (1997-12), pages 7803-7811, XP002139885 ISSN: 0021-9193 cited in the application
- C. SCHLEPER ET AL.: "Genomic analysis reveals chromosomal variation in natural populations of the uncultured psychrophilic archaeon Cenarchaeum symbiosum" J. BACTERIOL., vol. 180, no. 19, October 1998 (1998-10), pages 5003-5009, XP002139886 AM. SOC. MICROBIOL.,BALTIMORE,US; cited in the application
- BARNS SUSAN M ET AL: "Remarkable archaeal diversity detected in a Yellowstone National Park hot spring environment." PROCEEDINGS OF THE NATIONAL ACADEMY OF SCIENCES OF THE UNITED STATES OF AMERICA 1994, vol. 91, no. 5, 1994, pages 1609-1613, XP002139887 ISSN: 0027-8424
- J. KJEMS ET AL.: "Phylogenetic relationship amongst the hyperthermophilic archaea determined from partial 23S rRNA gene sequences" SYSTEM. APPL. MICROBIOL, vol. 15, no. 2, 1992, pages 203-208, XP000914637 FISCHER, STUTTGART, FRG
- HUBER R ET AL: "PYROBACULUM NEW-GENUS OF NEUTROPHILIC ROD-SHAPED ARCHAEBACTERIA FROM CONTINENTAL SOLFATARAS GROWING OPTIMALLY AT 100 C" ARCHIVES OF MICROBIOLOGY 1987, vol. 149, no. 2, 1987, pages 95-101, XP000914645 ISSN: 0302-8933
- M. KAEHLER AND G. ANTRANAKIAN : "Pyrobaculum islandicum family B DNA polymerase (polB3) gene, complete cds." EMBL SEQUENCE DATABASE, 26 January 2000 (2000-01-26), XP002139889 Hinxton, UK
- DATABASE BIOSIS [Online] BIOSCIENCES INFORMATION SERVICE, PHILADELPHIA, PA, US February 2000 (2000-02) KAEHLER MARKUS ET AL: "Cloning and characterization of a family B DNA polymerase from the hyperthermophilic crenarchaeon Pyrobaculum islandicum." Database accession no. PREV200000113650 XP002139890 & JOURNAL OF BACTERIOLOGY FEB., 2000, vol. 182, no. 3, February 2000 (2000-02), pages 655-663, ISSN: 0021-9193

## Description

The invention is related to a DNA polymerase obtainable from *Pyrobaculum islandicum* whereas the six conserved motifs indicative of family B DNA polymerases were contained in the *Pyrobaculum* islandicum DNA polymerase sequence and whereas this polymerase exhibits 3'-5'-exonuclease activity.

Archaea constitute the third domain of life, distinguishable from the domains Eukarya and Bacteria (Woese and Fox 1977). During the last two decades, many Archaea have been isolated and characterized. Hyperthermophilic Archaea, which are the deepest and shortest branches of the phylogenetic tree, can grow at temperatures up to 110°C (Stetter 1996). Thus, these organisms are excellent candidates to investigate their thermostable enzymes. While our knowledge of DNA replication in Eukarya and Bacteria is quite advanced (Kornberg and Baker 1992), only limited information on the replication of DNA in hyperthermophilic Archaea is available. It is still an open question how genomes can be maintained and duplicated at temperatures near or above 100°C.

More than 50 DNA polymerase genes from Eukarya, Bacteria and Archaea have been cloned and sequenced. Deduced from their amino acids sequences, these enzymes can be classified into four groups, represented by *Escherichia coli* DNA polymerase I (family A), DNA polymerase II (family B), DNA polymerase III (family C), and eucaryotic DNA polymerase β (family X) (Braithwaite and Ito 1993). The majority of archaeal DNA polymerases described so far belongs to the family B. A number of euryarchaeal DNA polymerase genes has been cloned and sequenced. The thermostable enzymes from several *Thermacoccus* and *Pyrococcus* strains have been successfully expressed, characterized and can be obtained commercially (Perler et al. 1996). Relatively little information is available on DNA polymerases from hyperthermophilic crenarchaeota. Uemori et al. (1995) described the cloning and characterization of two family B DNA polymerases from the hyperthermophilic crenarchaeon *Pyrodictium occultum.* Despite of the fact that three family B DNA polymerase genes have been detected in *Sulfolobus solfataricus* (Prangishvili et al. 1993; Datukishvili et al. 1996; Edgell et al. 1997), only one of these genes has been successfully expressed (Pisani et al. 1992).

DNA polymerases from hyperthermophiles play a major role in a variety of molecular biological applications, e.g. DNA amplification, sequencing, labelling or reverse transcription. The development of polymerase chain reaction (PCR; Mullis et al. 1986; Erlich et al. 1988) was one of the most important contributions to biotechnology in the last decade. The DNA polymerase I from the bacterium *Thermus aquaticus,* called *Taq* polymerase, was the first thermostable DNA polymerase applied in PCR. Due to the absence of a 3'-5'exonuclease activity, this enzyme is unable to excise mismatches and as a result, the base insertion fidelity is low (Longley et al. 1990; Keohavong and Thilly 1989). Furthermore, research has indicated that *Taq* polymerase has a thermal stability of not more than few minutes at 100°C. Several euryarchaeal DNA polymerases with much higher thermostability and an associated 3'-5'exonuclease activity, e.g. *Pfu* pol (Lundberg et al. 1991) from *Pyrococcus furiosus* or Vent pol (Cariello et al. 1991) from *Thermococcus litoralis,* have been described and are commercially available. However, *Taq* polymerase was not replaced by these polymerases mainly due to their low extension rates. Therefore, new DNA polymerases with enhanced thermostability, high base insertion fidelity and sufficient extension rates are still needed. Hyperthermophilic crenarchaeota could be a promising source for thermostable DNA polymerases with new properties. Additionally, the production of large quantities of recombinant enzymes would be of great value.

The anaerobic hyperthermophilic Archaeon *Pyrobaculum islandicum,* which belongs to the crenarchaeota, was isolated from an Icelandic hot spring (Huber et al. 1987). It was not possible to isolate polymerases from the native strain without undue experimentation because this strain is difficult to culture. Furthermore, it was not possible to amplify fragments of the B DNA polymerase gene of *Pyrobaculum islandicum* using conventional primers.

### PCR Primer Screening

In order to amplify fragments of the *Pyrobaculum islandicum* DNA polymerase, we performed a primer screening with various kind of primers. Archael family B DNA polymerase are difficult to align due to short, highly conserved domains separated by long stretches of low or no amino acid conservations. It seems to be particularly difficult to clone a new family B DNA polymerase gene from an archaeon which shows phylogenetically large distances to archaea from which polymerase sequences have been identified.

*Pyrobaculum islandicum* belongs to the crenarchaeal order Thermoproteales. When we started our investigations, only a few crenarchaeal polymerase genes from *Pyrodictium* and *Sulfolobus* species, and some polymerase gene sequences from the euryarchaeal *Pyrococcus* and *Thermococcus* species have been identified.

The PCR primer screening (using *Pyrobaculum islandicum* DNA as template) was started with the combination of several primers deduced from conserved *Thermococcus* and *Pyrococcus* polymerase sequences:

### forward primers

tcg1: 5'-CCGCAAATGATACTGGACACTGATTACATAAC-3' SEQ ID No. 5

pcw1: 5'-CCGCAAATGATTTTAGATGTGGATTACATAAC-3' SEQ ID No. 6

c1435: 5'-GAGCCAGAAAARGGKTTGTGGAAAA-3' SEQ ID No. 7

### reverse primers

tcg2: 5'-CCGCAATTAGGATTTTTTAATGTTAAGCCAGGAAG-3' SEQ ID No. 8

pcw2: 5'-CCGCAATTACCTCTTGAGCCATGCATCTAAGCCGG-3' SEQ ID No. 9

c1190: 5'-TCATAMGTTGCCYTWGCATCTTCCAT-3' SEQ ID No. 10

The primer pair tcgl/c1190 was successfully used before to amplify a *Thermococcus* polymerase gene fragment (Niehaus et al. 1997).

In contrast to the *Thermococcus polymerase* gene however, it was not possible to amplify any PCR products from *Pyrobaculum islandicum* showing identities to family B DNA polymerases.

Uemori et al. (1995) also used degenerated primers, deduced from highly conserved regions of family B DNA polymerases genes:

forward, 5'-(TA)(CG)(CG)(CT)T(CG)TAC CC(CG) (TA)(CG) ATC AT-3'

reverse, 5'-TC(TCAG) GT(AG) TC(TCAG) CC(AG) TA(AG) AT(TCAG) AC-3

A PCR with these primers and *Pyrobaculum islandicum* DNA as template was also not successful.

Therefore, it was necessary to design new degenerated primers. Additionally, it can be assumed that the use of degenerated primers, including deoxyinosit (I), may be a great advantage in amplifying new archaeal genes, because primers with deoxyinosit accept several degenerated tripletts.

The two available family B DNA polymerase genes from *Pyrodictium occultum* (Uemori et al. 1995; ass. no. D38574 and D38573) and one polymerase gene from *Sulfolobus solfataricus* (ass. no. Y08257) was chosen for an alignment in order to synthesize suitable primers for a primer screening.

Due to the degenerated triplett code, it was necessary to design degenerated primers, using deoxyinosin (I). The primers were deduced from highly conserved regions of family B DNA polymerases.

### forward primers

f1: 5'-CTG ACC TT(CT) AA(CT) GGI GA(CT)-3 SEQ ID No. 11

f2: 5'-CAT ATI GAC (CT)TI TA(CT) AAI TT-3' SEQ ID No. 12

f3: 5'-GTI (CT)TI GAT TT(CT) GCI (AT)(GC)I (CT)TI TA(CT) CC-3' SEQ ID No. 13

f4: 5'-ATI AAG GGI AA(AG) AAG TAT-3' SEQ ID No. 14

### reverse primers

r1a: 5'-(CT)TC IGG IGT (AG)TT IC(GT) (CT)TT (CT)TT IGC-3' SEQ ID No. 15

r1b: 5'-GAA IAG GTC IGT ATC ICC AT-3' SEQ ID No. 16

r2a: 5'-GAA IAG IGA (AG)TC IGT (AG) TC ICC (AG)T-3' SEQ ID No. 17

r2b: 5'-GAA IAG IGA GTC IGT ATC ICC AT-3' SEQ ID No. 18

Amultiple primer screening was performed with combinations of each forward with each reverse primer. A low annealing temperature of 48°C was used.

Only one PCR with the forward primer f3 and the reverse primer r2b resulted in the amplification of a PCR product, showing identities to archaeal family B DNA polymerases. This fragment was used as a probe to identify the complete polymerase gene, as described in Example 2. This result indicated that it is very difficult to design appropriate primers for the amplification of new crenarchaeal DNA polymerase gene fragments.

The subject of the present invention comprises the cloning procedure and sequence of a DNA polymerase gene from *Pyrobaculum islandicum.* The invention comprises a gene with SEQ ID No. 3. The subject of the present invention also comprises a B DNA polymerase from *Pyrobaculum islandicum.* The invention comprises a polymerase with a sequence according to SEQ ID No. 4. Furthermore, the expression, purification and characterization of the recombinant polymerase from *P. islandicum* is disclosed.

### Cloning and nucleotide sequencing of the DNA polymerase gene from P. islandicum

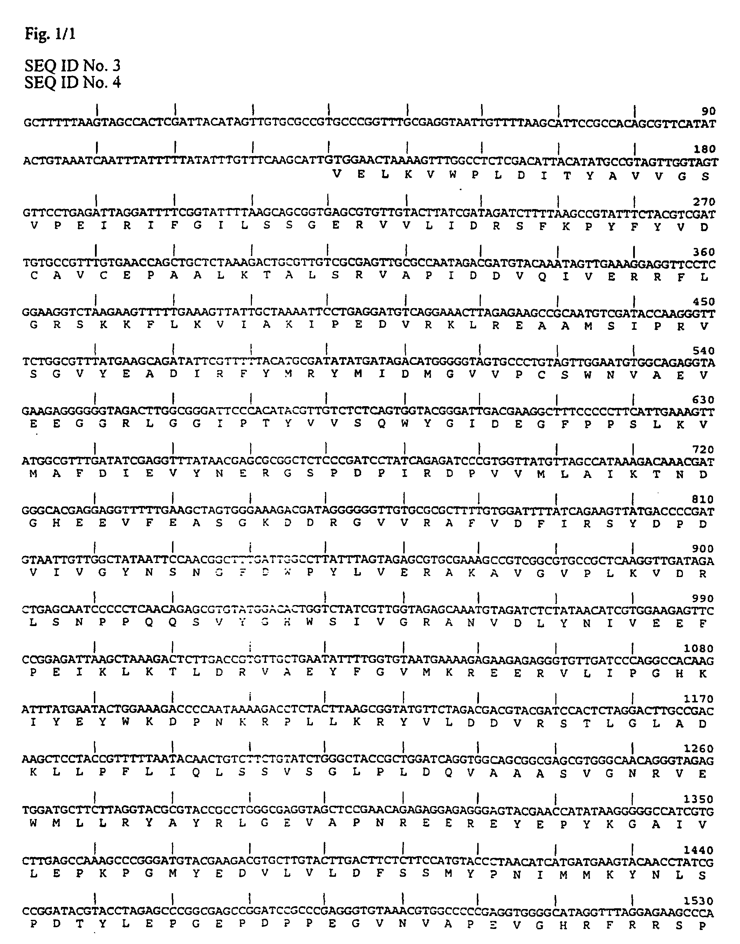

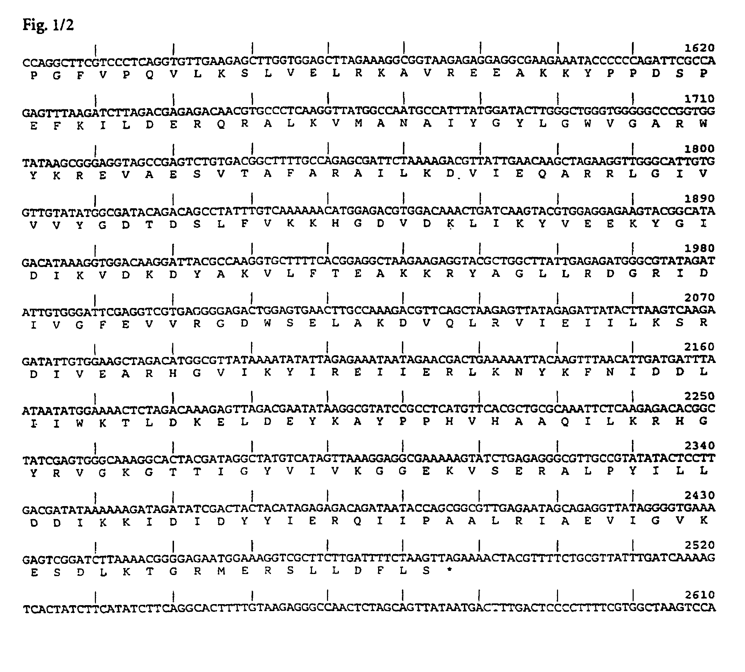

Based on the most conserved amino acid sequences found in archaeal α-like DNA polymerases, two degenerated primers according to SEQ ID No. 1 and SEQ ID No. 2 were designed. The PCR was performed with chromosomal DNA from *P. islandicum,* and we were able to obtain an amplificate of 445 bp. The PCR product was sequenced, and the sequence showed the expected homology to known archaeal polymerase regions in a BLAST alignment. As an important result of the present invention, known archaeal DNA polymerase sequences can be successfully used to design novel degenerated primers in order to find new DNA polymerase genes from hyperthermophilic archaea. Therefore, the cloning procedure using degenerated primers according to SEQ ID No. 1 and SEQ ID No. 2 is also subject of the present invention. The PCR product was then used as a probe to obtain the whole DNA polymerase gene. In southern blot hybridizations, *P. islandicum* DNA fragments in a size of 6 to 7.5 kbp, digested with SacI, were chosen to be cloned in a cosmid vector system. An open reading frame consisting of 2356 bp and coding for a protein with 784 amino acids (Fig. 1) was detected on an insert of a cosmid clone, which was identified by colony filter hybridization. The open reading frame started with GTG, coding for valin. It has been shown that some archaeal proteins (approx. 25 %) appear to start at GTG codons (Dennis 1997). For example, Schleper et al. (1997; 1998) described several open reading frames in the crenarchaeote *Cenarchaeum symbiosum,* including that of a family B DNA polymerase gene, starting with GTG instead of ATG. In order to examine the putative transcription and translation start region of the DNA polymerase gene from *P. islandicum,* a 5'RACE PCR was performed and the amplified cDNA was sequenced. The sequence of the mRNA started one or two nucleotides upstream of the determined start codon (data not shown).

The DNA polymerase sequence was aligned with those of all archaeal DNA polymerases available in public databases. The six conserved motifs indicative of family B DNA polymerases (Braithwaite and Ito 1993) were identified in the *P. islandicum* sequence, including those residues that are invariant in all DNA polymerases and have been shown to be functionally important. Also, the three 3'-5'exonuclease motifs (Blanco et al. 1991) were localized in the sequence. In a multiple alignment performed with the ClustalW software program (Higgins, EMBL, Heidelberg, Germany), the DNA polymerase sequence showed highest identities to one of the two family B DNA polymerases from *Pyrodictium occultum* (polB; accession. no. D38574), a family B DNA polymerase from *Archaeoglobus fulgidus* (accession no. AE001070) and the *Pfu* polymerase (accession no. D12983), sharing 51%, 37% and 32% identical residues, respectively.

### Expression and purification of P. islandicum DNA polymerase

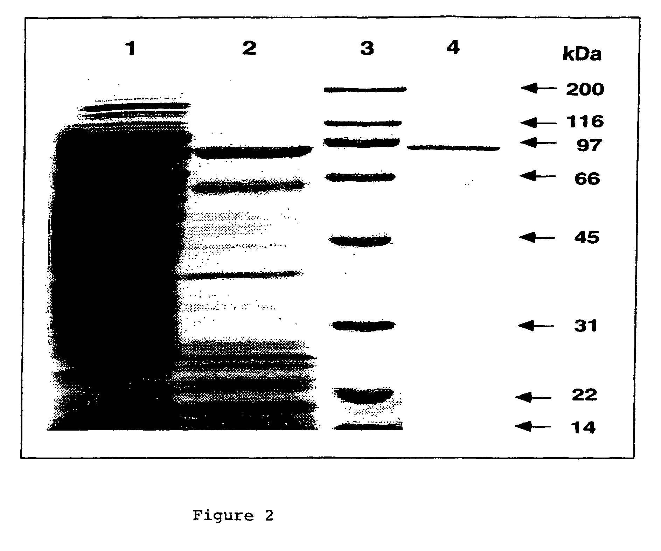

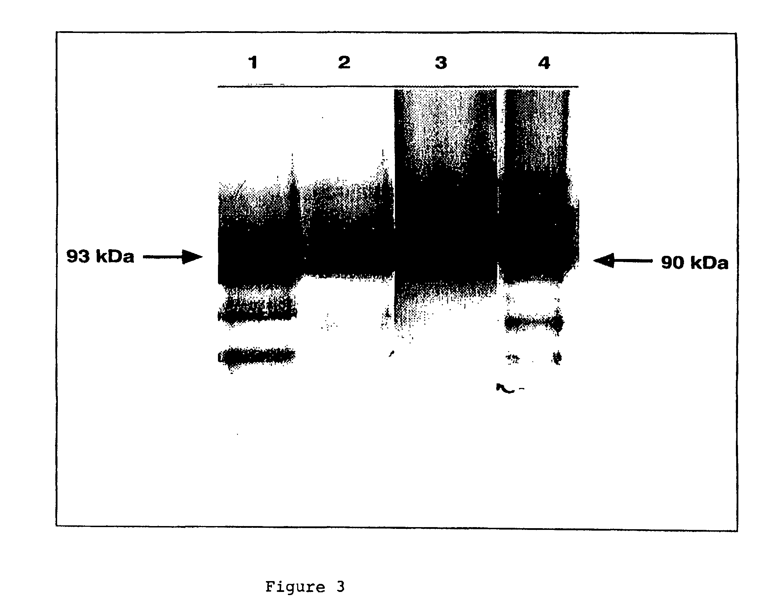

After determining the sequence, we cloned the gene into vector pASK-IBA 3 and expressed the polymerase as a fusion protein with a C-terminal Streptactin-binding oligopeptide. The recombinant DNA polymerase was purified to near homogeneity by the combination of a heat denaturation step and an affinity chromatography on Streptactin. Starting with 1000 ml of *E. coli* BL21 culture, we purified 1 mg of recombinant DNA polymerase with a specific activity of approx. 2000 U/mg preparations of recombinant DNA polymerase. Attempts to purify the polymerase without addition of protease inhibitors to the buffer W resulted in the appearance of many small contaminants as judged by SDS-PAGE (data not shown). The purity of the DNA polymerase in each fraction were checked by SDS-PAGE (Fig. 2). The fusion protein was copurified with a small amount of a ca. 70 kDa protein as a contaminant. The activity gel illustrated that the purified protein of approx. 90 kDa in the Streptactin preparation possesses DNA polymerase activity (Fig.3). A small activity was also detectable at approx. 70 kDA, indicating that the copurified protein was a proteolytic degradation product of the recombinant polymerase. Additionally, in crude extract from P. *islandicum,* we were able to detect corresponding DNA polymerase activity in a protein band of approx. 90 kDa.

Subjects of the present invention are also mutants of the inventive B DNA polymerase and DNA sequencing encoding these mutants. These mutants may exhibit e.g. reduced proofreading acitivty according to mutations in the highly preserving amino acid regions for this exonuclease activity (ExoI, ExoII and ExoIII) e.g.ExoI region contains an X1DX2EX3 motif, and the amiono acids D (aspartic acid) and E (glutamic acid) are known to be essential for the exonuclease activity for B typ polymerases.

### Biochemical properties

To determine the biochemical properties of the purified recombinant DNA polymerase, we carried out the non-radioactive assay described in Materials and Methods under different reaction conditions.

The optimal conditions for DNA polymerase activity were tested at 75°C in the presence of activated calf thymus DNA as a template. As shown in Fig. 4, the polymerase was most active at pH 7.3. The optimal temperature for activity was not detectable because activated DNA was not stable above 80°C. The enzyme required an extremely low concentration of divalent cations for activity. Concentrations above the optimum of 0.5 mM MgCl₂ inhibited the polymerase (Fig. 5). No activity was detected in the absence of divalent cations. As illustrated in Fig. 6, monovalent potassium ions strongly inhibited the DNA polymerase. In the presence of 100 mM KCl, only 7% of the residual activity was detected.

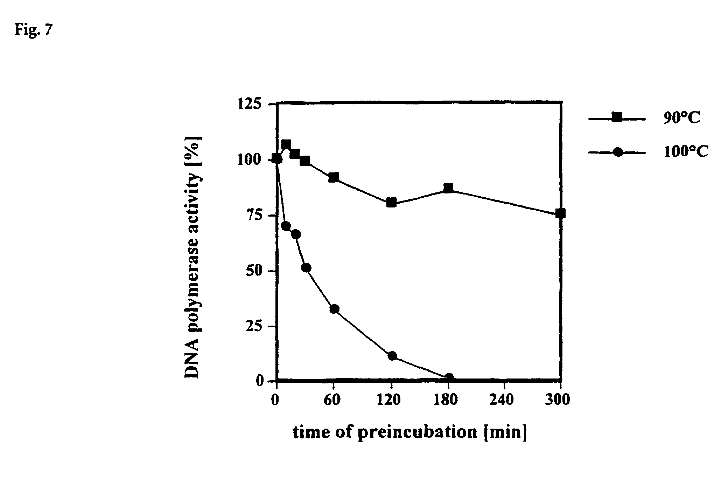

To determine the thermostability of the DNA polymerase, a preincubation of the enzyme in 50 mM Tris-HCl pH 7.2 was performed at 90 and 100°C, following by an incubation at 75°C under standard assay conditions. Half-life times of 30-40 min at 100°C and > 10 h at 90°C have been detected (Fig. 7).

The influence of representative inhibitors on the DNA polymerase activity was investigated (Tab. 1). The sulfhydryl blocking reagent N-Ethylmaleimid (NEM) strongly inhibited the DNA polymerase activity. In the presence of 1 mM NEM, 40% residual activity were detected. A total loss of activity was detected after a preincubation with 5 mM NEM. The sensitivity of the enzyme to the tetracyclic diterpenoid aphidicolin, which competes with each dNTP for binding to DNA polymerase, was also investigated. The DNA polymerase was inhibited by a comparable amount of aphidicolin that inhibits polB from *Pyrodictium occultum* and *Pfu* polymerase (Uemori et al. 1995). In the presence of 500 and 2000 µM aphidicolin, we detected 55 and 21% residual activity for the *P. islandicum* DNA polymerase. A very low sensitivity of the polymerase to ddGTP was detected. No inhibition was measured at a ddGTP/dGTP ratio of 2, and at a ddGTP/dGTP ratio of 10, approx. 80% of residual activity could be detected.

In order to determine associated exonuclease activity in the DNA polymerase from *P. islandicum,* we first incubated *BamHI* digested pUC 19 DNA with the purified enzyme in the absence of dNTP. A significant degradation after 2 h at 75°C was detected on an agarose gel (data not shown). No degradation was measured at 37°C, indicating that no exonuclease contamination from the *E. coli* BL21 cells was present in the enzyme preparation after heat-treatment and purification on Streptactin Sepharose.

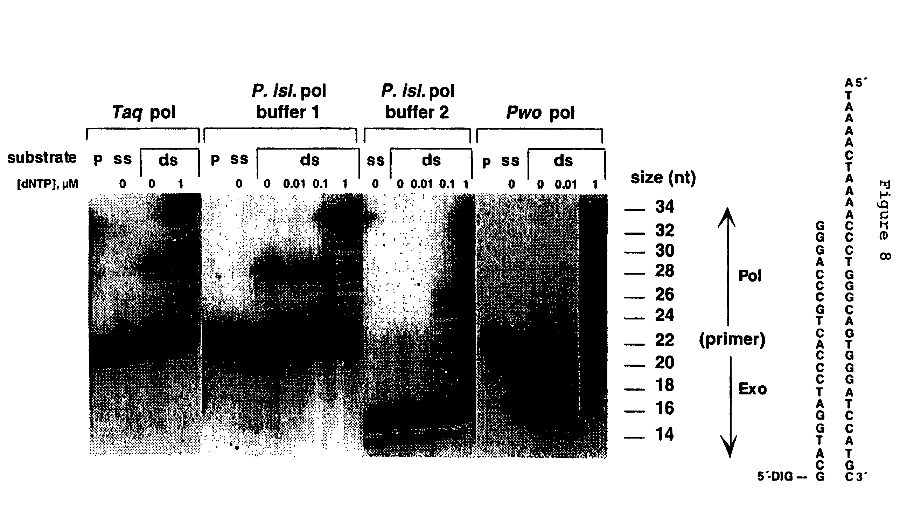

The exonuclease activity was quantified in a standard assay, based on the release of ³H-labeled nucleotides from a sonicated calf thymus DNA template. The release of nucleotides after an incubation of the template with 1.5 U purified polymerase at 65°C was comparable to that caused by other archaeal proofreading DNA polymerases. 104 cpm/10 min were measured after 4 h incubation. In comparison, 15 U (10-fold higher concentration) of *Taq* polymerase caused a cpm/10 min of 150-250, and the same amount of proofreading polymerases such as *Pfu* polymerase caused 1000-3000 cpm/min No release of nucleotides was detected after incubations at 37°C, indicating that no E. coli exonuclease activity interferes with the test. The detected exonuclease activity of the *P. islandicum* DNA polymerase corresponds to the three 3'-5'exonuclease domains identified within the deduced DNA polymerase sequence (Blanco et al. 1991), indicating that this kind of exonuclease activity and not a 5'-3'exonuclease activity was measured.
Additionaly to the above described exonuclease activity assay 3'-5' exo-nuclease activity was tested on single strands as described below. To investigate if a 3'-5' exonuclease activity on single- and double-stranded DNA could be detected, we used a 5'-DIG-labeled primer and a primer/template complex. 3'-5' exonuclease activity was shown by the degradation of the primer after incubation with the DNA polymerase at 70°C for 1 h. As indicated in Fig. 8, the rate of *P. islandicum* DNA polymerase-associated 3'-5' exonuclease activity strongly depends on the pH of the reaction buffer. Using the optimal polymerase buffer (pH 7.3) the degradation of the primer by the enzyme from P. *islandicum* was significantly lower than with *Pwo* polymerase, whereas in buffer 2 (pH 8.6) the value for exonuclease activity was comparable to that of *Pwo* polymerase. Both on single-stranded primer and double-stranded primer/template the DNA polymerase from *P. islandicum* showed 3'-5' exonuclease activities in the absence of dNTPs. Even in the presence of 0.01 and 0.1 µM dNTPs a *Taq* polymerase did not show any degradation of the primer, neither on single-stranded nor on double-stranded DNA substrate (Fig. 8). As expected for DNA polymerases with 3'-5' exonuclease activity, the degradation of the primer/template substrate by the DNA polymerases from *P. islandicum* and *P. woesei* was reduced in the presence of higher amounts (1 µM) of dNTPs. Neither degradation nor elongation of the primer by the DNA polymerase from *P. islandicum* was detectable when the assay was performed at 37°C (data not shown). This result clearly indicates that the enzyme preparation did not contain a contaminating exonuclease activity from *E. coli* which could interfere with the test.

### Applications

In this invention, we present the cloning procedure of a DNA polymerase gene and some biochemical properties of a new thermostable DNA polymerase from *Pyrobaculum islandicum.* The major uses of thermostable DNA polymerases are for in vitro amplification of DNA fragments and for determination of DNA sequences. *Taq* DNA polymerase is still used in many cases, but its fidelity is very low due to the absence of a 3'-5' exonuclease activity (Keohavong and Thilly 1989; Longley et al. 1990). Additionally, *Taq* polymerase has a thermostability of not more than few minutes at 100°C.

The DNA polymerase presented in this invention possesses a high thermostability at 90 and 100°C. It has to be investigated whether the presence of stabilizers such as nonionic detergents or bovine serum albumin (BSA) can even lead to an increased thermostability. The positive influence of the nonionic detergent octoxynol (commonly known as TRITON X-100) and BSA on the thermostability of DNA polymerases from *Pyrococcus furiosus* and *Thermococcus litoralis* has been described (EP Application no. 92118965 and 91303787.5). The thermostable DNA polymerase from *Pyrobaculum islandicum* is not irreversible inactivated when subjected to the elevated temperatures for the time interval which is necessary to effect denaturation of double-stranded DNA, a key step in the PCR process. As a result, the enzyme may provide additional advantages over the previously characterized thermostable enzymes. Higher temperatures up to 100°C may be required, e.g. as the GC composition of the DNA template is increased. For target sequences, denaturation temperatures over 95°C are needed. For these purpose, solvents such as glycerol or DMSO are included in PCR in order to partially destabilize the duplex DNA (Wong et al. 1991; Korge et al. 1992). These agents, in addition to destabilizing duplex DNA will affect primer stability, can inhibit activity and decrease thermostability of polymerases. In contrast, simply raising the denaturation temperature up to 95 or 100°C with *Pyrobaculum islandicum* DNA polymerase in an otherwise standard PCR can facilitate complete strand separation of PCR product eliminating the need for DNA helix destabilizing agents.

Furthermore, there is still a demand to obtain and produce a purified DNA polymerase having 3'-5'proofreading exonuclease activity with even high thermostability. The *P. islandicum* DNA polymerase with 3'-5' proofreading exonuclease activity has a substantially lower base incorporation error rate when compared with a non-proofreading DNA polymerase.

PCR performed with *P. islandicum* DNA polymerase. Thermostable DNA polymerases are particularly used for *in vitro* amplification of DNA fragments by PCR and for DNA sequencing. Due to the associated 3'-5' exonuclease activity, archaeal DNA polymerases offer the possibility to amplify DNA fragments with high fidelity (low mutation frequency). To date, all archael DNA polymerases used in PCR are derived from Euryarchaeota. No reports are available concerning the successful application of crenarchaeal DNA polymerases in PCR.

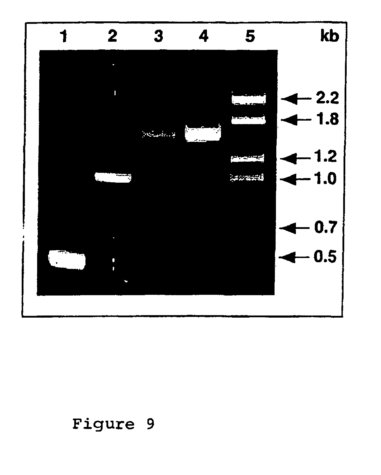

To verify the suitability of the DNA polymerases from *P. islandicum* in DNA amplification, PCR was performed using 1 U of enzyme and several primer combinations. As shown in Fig. 9, up to 1500 bp of a λ DNA template could be amplified. This is the first report on the possible use of a crenarchaeal DNA polymerase in PCR.

As above stated a method of a cloning procedure for B DNA polymerases using degenerated primers according to SEQ ID No. 1 and SEQ ID No. 2 is also subject of the present invention. Furthermore, a method of the amplification of fragments of a B DNA polymerase using primers according to SEQ ID No. 1 and 2 is subject of the present invention. The invention also includes the above mentioned methods if the following variants of the primers with SEQ ID No. 1 and 2 are used:
- primers wherein tripletts are added or omitted, especially if 1-3 tripletts are omitted and 1-5 tripletts are added
- primers wherein one or more of the inosins shown in SEQ ID No. 1 or SEQ ID No. 2 are resubstituted by a naturally occurring base
- primers wherein one or more naturally occurring bases are substituted by inosin in addition to the substitutions according to SEQ ID No. 1 or SEQ ID No, 2, preferentially a naturally occurring base is substituted by inosin which is at the third position of a triplett.

**Table 1:**

| | | |
|---|---|---|
| Influence of inhibitors on the DNA polymerase of P. *islandicum* The influence of different inhibitors was detected at standard assay conditions (pH 7.5; 0.5 mM MgCl₂; 0 mM KCl; 75°C) using 0.5 U purified DNA polymerase. A preincubation of the enzyme with N-Ethylmaleimid was performed at 90°C for 20 min. | | |

| Inhibitor | Concentration | DNA polymerase activity |
|---|---|---|
| | mM | % |
| N-Ethylmaleimid | 0 | 100 |
| | 1 | 40 |
| | 2 | 30 |
| | 5 | 0 |
| | µM (µg/ml) | |
| Aphidicolin | 0 (0) | 100 |
| | 50 (17) | 100 |
| | 200 (68) | 70 |
| | 500(170) | 55 |
| | 2000(680) | 21 |
| ddGTP | ddGTP/dGTP = 5 | 95 |
| | 10 | 80 |
| | 20 | 53 |

### Example 1:

### Strains, enzymes, vectors and chemicals

*Pyrobaculum islandicum* DSMZ4184 (Huber et al. 1987) was obtained from the Deutsche Sammlung von Mikroorganismen und Zellkulturen, GmbH (DSMZ; Braunschweig, Germany).

Magnesium cultures of *E. coli* DH5α cells were included in the Expand Cloning Kit (Boehringer Mannheim). *E. coli* BL21 cells were obtained from Novagen Inc., Madison, USA.

Enzymes and kits for manipulations of DNA were obtained from Boehringer Mannheim or, if mentioned, from other customers. Kits for purification and gel extraction of DNA were products of QIAGEN (Hilden, Germany). A kit for preparation of plasmid and cosmid DNA was obtained from BioRad (Munchen, Germany).

The expression vector pASK-IBA3, the inductor anhydrotetracycline and the Streptactin Sepharose columns were obtained from IBA, Göttingen, Germany.

Materials for the characterization of DNA polymerase were purchased from Boehringer Mannheim. N-Ethylmaleimid was obtained from Serva. Other chemicals for the preparation of media and buffers were obtained from Difco (Detroid, Wi), Serva (Heidelberg, Germany), Sigma (St. Louis, Mo.) and Merck (Darmstadt, Germany).

### Isolation of DNA

*P. islandicum* was grown anaerobically at 98°C in 1.5 1 Medium 390 as recommended by DMSZ. Cells were harvested, washed with TE buffer (10 mM Tris-HCl pH 8.0, 1 mM EDTA) and suspended in 3 ml 20% sucrose containing 10 mM Tris-HCl pH 8.0. Triton X-100 was added to a final concentration of 0.3%. After incubation at 20°C for 1 h, 3 ml of SET buffer (150 mM NaCl, 20 mM Tris-HCl pH 8.0, 1 mM EDTA), 0.32 ml of 10% sodium dodecyl sulfate and 0.16 ml of Proteinase K (10 mg/ml) were added. The mixture was incubated for 1 h at 37°C. After subsequent phenol-chloroform extraction, DNA was recovered by ethanol precipitation (Maniatis et al. 1982).

### Example 2:

### PCR and preparation of probes

For the first PCR, we designed two degenerated primer based on the conserved regions I and II of α-like DNA polymerases (Wang et al. 1989).

### The forward primer was:

SEQ ID No. 1: 5′-GTI(CT)TIGATTT(CT)GCI(AT)(GC)I(CT)TITA(CT)CC-3′,

and the reverse primer was: SEQ ID No. 2: 5′-GAAIAGIGAGTCIGTATCICCAT-3′, using deoxy-inosin (I), which is complementary to all four nucleotides. Primers were custom synthesized by ARK Scientific, GmbH Biosystems (Darmstadt, Germany). Approx. one nanogram *of P. islandicum* DNA and 100 pmol of each primer were added to a PCR reaction mix containing 200 µM deoxyribonucleoside triphosphates, the Expand^{TM} High Fidelity buffer system and 2 U of Expand^{TM} High Fidelity enzyme (Boehringer Mannheim). After an initial denaturation step at 94°C for 2 min, 30 cycles with a temperature profile of 10 sec at 94°C, 30 sec at 46°C and 90 sec at 68°C were performed with a DNA thermal cycler (GeneAmp PCR System 2400; Perkin-Elmer, Weiterstadt). Following the last cycle, the samples were incubated for additional 7 min at 68°C, to ensure completion of the extension step. The PCR product was purified and sequenced. Other PCR reactions were performed under the same conditions with different annealing temperatures and elongation times. The same PCR reaction mix, also containing the template and primer described above and 200 µM dATP, dGTP and dCTP, but 190 µM dTTP and 10 µM DIG-11-dUTP (digoxigenin-labeled dUTP, alkali-stable; Boehringer Mannheim) was used to prepare a non-radioactive probe.

### Genomic southern blot hybridization

*P. islandicum* DNA was digested over night with several restriction enzymes and resolved on an 0.7% agarose gel. The restricted DNA was transferred to a positively charged nylon membrane. The DIG-labeled probe described above was used to perform hybridizations at 68°C, and a three-step process, including membrane blocking, incubating with anti-DIG-antibody, Fab fragments and a reaction of the antibody-conjugated alkaline phosphatase with the chemiluminescent substrate CDP-Star^{TM} (Boehringer Mannheim) was performed to detect the sites of the hybridized probe.

### Cosmid cloning

Following the southern blot, the restriction enzyme SacI was chosen to perform a preparative digestion of *P. islandicum* DNA. Restricted DNA fragments with a size between 6 and 7.5 kbp were extracted from an agarose gel and cloned using the Expand^{TM} Cloning Kit (Boehringer Mannheim). The fragments were blunt-ended and ligated into the cosmid vector, and the constructs were packed into bacteriophages. An *E. coli* DH5α magnesium culture was subsequently infected, and positive clones were selected by the vector-mediated ampicillin resistance. A cosmid clone containing the DNA polymerase gene was identified by colony filter hybridization (DIG System User's Guide for Filter Hybridization), using the probe described. Cosmid DNA from a positive clone was prepared and sequenced.

### DNA sequencing

Purified PCR products and cosmid DNA preparations were custom sequenced by SeqLab (Göttingen, Germany). The nucleotide sequences were determined by the dideoxy chain termination method (Sanger et al. 1977). All DNA samples were sequenced using ABI PRISM^{TM} BigDye terminators, and analyzed by automated sequencers with a sequence analysis software (Applied Biosystems).

### Example 3:

### RNA isolation and determination of transcription start

In order to examine a putative transcription start region of the detected DNA polymerase gene, RNA from freshly cultivated P. *islandicum* cells was prepared, following a protocol written by DiRuggiero and Robb (1995), and a 5'RACE PCR was performed, using the 5'/3'RACE Kit (Boehringer Mannheim). For first-strand cDNA synthesis, cDNA amplification and control PCR, reverse primer deduced from the gene sequence were used. The amplified cDNA was gel extracted and directly sequenced.

### Example 4:

### Expression and purification of P. islandicum DNA polymerase

The DNA polymerase gene was expressed in *E. coli* using the vector pASK-IBA 3 (IBA, Göttingen). The expression cassette of this vector is transcriptionally controlled by the chemically inducible tetracycline promotor (Skerra 1994). For convenient purification of the recombinant protein, the vector contained a C-terminal, nine amino acids coding sequence, called Strep-tag. The Strep-tag allows to purify the resulting fusion protein in an affinity chromatography step on Sepharose-coupled streptavidin or Streptactin (Schmidt and Skerra 1994; Voss and Skerra 1997). Two primers, Exforw-*Bsa*I (5'-GCTGATGGTCTCGAATGGAACTAAAAGTTTGGCCTCT-3') and Exrev-*Bsa*I (5'-GCTGATGGTCTCCGCGCTACTTAGAA.A.ATCAAGAAGCGACCT-3') were used to amplify the polymerase gene from chromosomal *P. islandicum* DNA. The forward primer was designed such that a restriction site for *Bsa*l was generated and the start codon, coding for valin, was removed. The recombinant fusion protein started with the vector-coded methionin. The reverse primer was designed such that a restriction site for *Bsa*I was generated and the stop codon of the polymerase gene was removed. The PCR product was purified by gel extraction, cleaved with *Bsa*I*,* again gel extracted and ligated into BsaI-restricted pASK-IBA 3 vector. Expression of the polymerase gene was performed in *E. coli* BL21. Transformed cells were cultivated at 37°C in 1000 ml of LB medium containing 100 µg ampicillin per ml. At an optical density of 0.5 at 600 nm, cells were induced by the addition of 100 µl anhydrotetracycline (2 mg/ml in dimethyl formamide; final concentration 200 µg/l), and the culture was incubated for a further 15 h. After cooling, the cells were harvested by centrifugation at 4°C, washed with buffer W, countering 50 mM Tris-HCl pH 8.0, 1 mM EDTA and the Complete mini, EDTA-free protease inhibitor cocktail (Boehringer Mannheim), and resuspended in 5 ml of buffer W. The cells were disrupted by sonication, and after centrifugation, the crude extract was heat treated at 80°C for 20 min and centrifuged again. The sample was applied to a Streptactin ready-to-use Sepharose column (1 ml), equilibrated with 5 ml buffer W. After washing the column twice with 5 ml buffer W, elution of the bound protein was effected by the stepwise application of 10 ml buffer W containing 2.5 mM desthiobiotin (Sigma). Active fractions were pooled and dialyzed against 500 volumes of 50 mM Tris-HCl pH 7.2 and 10% glycerol.

### Example 5:

### Polymerase activity assay

A non-radioactive colorimetric enzyme immunoassay (DNA Polymerase Assay, non-radioactive; Boehringer Mannheim) was performed, based on the incorporation of digoxigenin- and biotin-labeled dUTP, instead of radio-labeled nucleotides as used in the standard DNA polymerase assay, into the same DNA. As Suzuki et al. ( 1993) demonstrated, the results of a chemoluminescent enzyme-linked immunoassay, following the same principle as the test described here, displayed comparable results to the standard isotopic assay. The detection and quantification of the synthesized DNA followed the sandwich ELISA protocol, as recommended by the manufacture. Unless otherwise mentioned, the standard reaction mixture contained, in a 100 µl volume, 50 mM Tris-HCl pH 7.2,0.5 mM MgCl₂, 60 µg BSA, 0.36 µM DIG-11-dUTP, 18 nM Biotin-11-dUTP, 18 µM each dNTP, 0.27 µg DNaseI-activated calf thymus DNA as a template, and the DNA polymerase sample. The reaction mix was incubated at 75°C for 1 h. *Taq* DNA polymerase was used as a standard, with 50 mM Tris-HCl pH 7.9, 5 mM MgCl₂ and 50 mM KCl in the reaction mix. One unit was defined as the amount of enzyme required to incorporate 10 nmol of dNTP into an acid-insoluble form at 75°C in 30 min In order to determine the thermostability of the DNA polymerase from *P. islandi*cum, preincubations were performed in a reaction mix without BSA, labeled or non-labeled dNTP and template-DNA, following by the addition of the missing components and standard incubation of 1 h at 75°C. For the determination of the biochemical properties of DNA polymerase, all reaction mixtures contained 0.5 U of enzyme.

### Example 6:

### DNA polymerase activity gel

The detection of DNA polymerase activities on SDS polyacrylamid gels (SDS-PAGE) was performed according to a modified version (Niehaus et al. 1997) of the method described by Spanos and Hübscher (1983). Sample concentrations of one to five units of each DNA polymerase were run on SDS-PAGE (10%). Sample preparation and gel electrophoresis was carried out as described by Laemmli (1970). The running gel additionally contained 150 µg/ml DNAseI activated calf thymus DNA as template for the polymerases. DNA polymerases from *Thermus aquaticus* (*Taq* polymerase) and *Pyrococcus woesei* (*Pwo* polymerase) were purchased from Boehringer Mannheim. SDS was removed by immersing the gel 4 times in 50 mM Tris-HCl pH 7.2, 3 mM β-Mercaptoethanol, I mM EDTA, 5% glycerol. Renaturation of the proteins was performed at 4°C over night using 1 mM MgCl₂ and 1 µM dATP added to the same buffer. The gel was then incubated in activity buffer containing 50 mM Tris-HCl pH 7.2, 3 mM β-Mercaptoethanol, 1 mM DTT, 1 mM MgCl₂, 5% glycerol, 8 µM dGTP,dATP and dCTP, 4 µM dTTP and 5 µM DIG-11-dUTP, and incubated at 70°C for 4 h. DNA was transferred to a nylon membrane and incorporated DIG-11-dUTP was detected immunologically as described earlier.

### Example 7:

### Assay for exonuclease activity

To analyze exonuclease activity, we incubated 1.5 U purified polymerase with 1 µg of a ³H-labeled DNA template, which was digested by sonication. The released ³H-labeled nucleotides were detected as cpm/10 min on a scintillizer after 4 h incubation. Incubations were carried out at 65°C and 37°C in the buffer (10 mM Tris-HCl, 1 mM DTT, 50 mM NaCl, 10 mM MgCl₂) normally used in this test.

### Example 8:

### Assay for 3'-5'-exonuclease activity on single strands

For 3'-5' exonuclease activity on single-strands, the degradation of a synthetic primer (22-mer), 5'-labeled with digoxigenin for immunological detection, was analyzed. 3'-5' exonuclease activity on double-strands was measured using the same primer, hybridized to a template (34-mer). The sequences of primer and template are illustrated in Fig. 8. The assay principle was described previously (Niehaus, 1997 #77]. 0.5 pmol of the labeled substrate and 0.1 U of purified DNA polymerase were incubated in 10 µl of different buffers without and with increasing amounts of dNTPs (up to 1 µM) at 70°C for 1 h. To ensure that no contaminating exonuclease activity from *E. coli* was measured, the assay was further carries out at 37°C. The optimal DNA polymerase buffer containing 50 mM Tris-HCl pH 7.3 and 0,5 mM MgCl₂ was used as well as a buffer containing 50 mM Tris-HCl pH 8.6 and 0.5 mM MgCl₂. The reaction was stopped on ice with formamide buffer, and the samples were electrophoresed on a 12% polyacrylamide sequencing gel containing 8 M urea in TBE buffer. The degradation or polymerization products were visualized by immunological detection. *Pwo* polymerase in a buffer containing 10 mM Tris-HCl pH 8.9, 25 mM KCl, 2 mM MgSO₄ and 5 mM (NH₄)₂SO₄ and *Taq* polymerase in activity buffer described before were used as controls.

### Example 9:

### PCR reactions with P. Islandicum

PCR reactions with *P. islandicum* DNA polymerase were carried out with 1 U of enzyme in the following buffer: 15 mM Tris-HCl pH 8.6, 12,5 mM KCI, 2.5 mM (NH₄)₂SO₄, 1.25 mM MgCl₂, 20 µg/ml BSA. 10 ng of λ DNA was used as template. The forward primer 5'-GATGAGTTCGTGTCCGTACAACT-3' was combined with the following reverse primers: 5'-GGTTATCGAAATCAGCCACAGCG-3' for amplification of 500 bp, 5'-GTTAACTTTGATTCTGGCCTGCG-3' for amplification of 1000 bp and 5'-GTGAGATAAACGGCAACTGCCGG-3' for amplification of 1500 bp. PCR cycles were carried out as described before with a temperature profile of 10 s at 94°C, 30 s at 56°C and 1.5 min at 75°C. Expand High Fidelity enzyme was used as a control under PCR conditions as recommended by the manufacturer.

### Figure Legend

- **Fig. 1:**: **Nucleotide sequence of the polymerase gene and deduced amino acid sequence of the family B DNA polymerase from**
***P. islandicum***
The nucleotides are numbered from the 5' site. The translation start site is at no. 131, the stop site at no. 2486.

- **Fig. 2:**: **SDS Page (10%) of enzymatic fractions during purification of recombinant DNA polymerase from *P. islandicum***
Lanes: 1, crude extract; 2, crude extract after heat treatment (20'80°C); 3, molecular weight marker (broad range, BioRad); 4, enzyme preparation after chromatography on Streptactin. Molecular weights are indicated.
- **Fig. 3:**: **DNA polymerase activity gel**
Lanes: *1, Taq* polymerase (5 U); 2, *P. islandicum* DNA polymerase enzyme preparation after chromatography on Streptactin (2 U); 3, crude extract from *P. islandicum;* 4, *Pwo* polymerase (2.5 U).

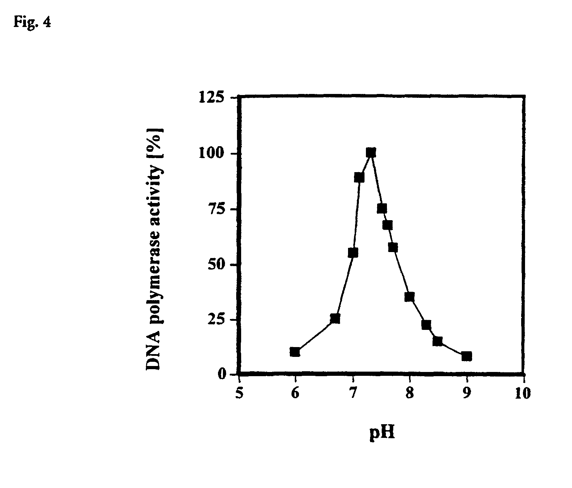

- **Fig. 4:**: **Optimal pH for the DNA polymerase activity**
The pH optimum was detected in a standard assay at 75°C with 0.5 mM MgCl₂.

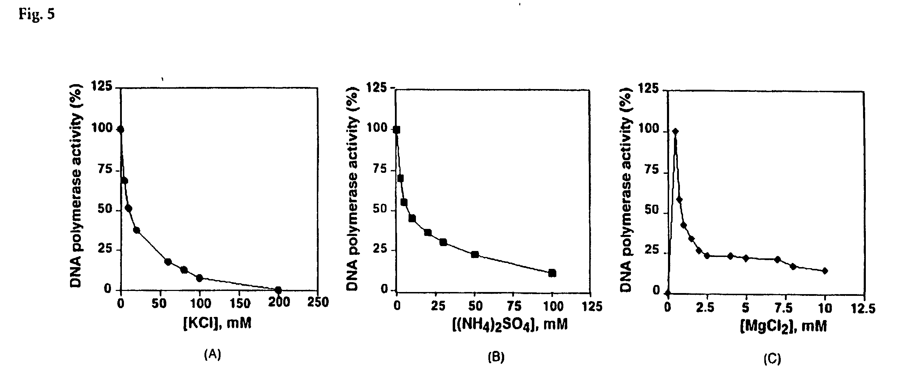

- **Fig. 5:**: **Optimal concentration of KCI, (NH**_{**4**}**)**_{**2**}**SO**_{**4**} **andMgCl**_{**2**} **for the DNA polymerase activity** The optimal concentration of MgCl₂ was measured in a standard assay at 75°C and pH 7.3. The standard assay was carried out with 0.2 U of purified DNA polymerase inthe presence of various concentrations of KCI, (NH₄)₂SO₄ and MgCl₂.

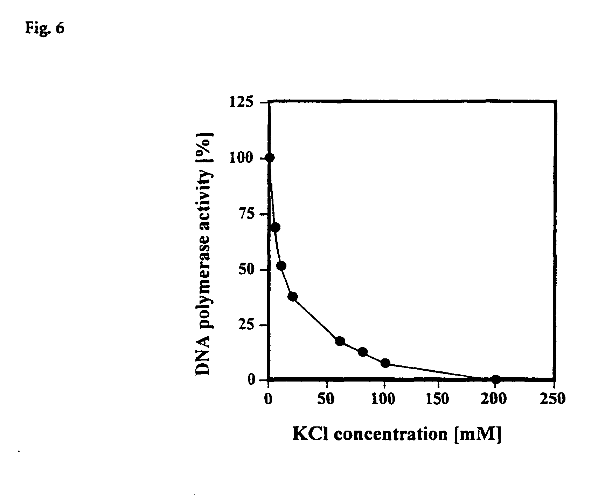

- **Fig. 6:**: **Influence of KCl on the DNA polymerase activity**
The influence of monovalent potassium ions was detected in a standard assay at 75°C, pH 7.3 and with 0.5 mM MgCl₂.
- **Fig. 7:**: **Thermostability of the recombinant DNA polymerase**
To estimate the thermostability of the DNA polymerase, the enzyme was preincubated without BSA, nucleotides and template DNA at 90 and 100°C, following by the addition of all missing compounds and a standard incubation at 75°C, pH 7.3 and with 0.5 mM MgCl₂.
- **Fig. 8:**: Single- and double-strand dependent 3'-5' exonuclease activity of DNA polymerases from *Pyrobaculum islandicum* (*P. isl.* pol), *Thermus aquaticus* (*Taq* pol) and *Pyrococcus woesei* (*Pwo* pol). The assay was carried out, as described in Materials and Methods, at 70°C for 1 h with 0.1 U of each polymerase. A digoxigenin-labeled primer as a single-stranded substrate (ss) and a primer/template complex as a double-stranded substrate (ds) were used. Concentrations of dNTP (µM) in each reaction are indicated. The reaction products were separated on a 12% polyacrylamide gel containing 8 M urea and visualized by immunological detection. The sizes (nucleotides, nt) of the non-degraded primer and the reaction products are indicated. Lane P, digoxigenin-labeled primer in buffer without dNTPs; buffer 1:40 mM Tris-HCl pH 7.3, 0.5 mM MgCl₂; buffer 2:50 mM Tris-HCl pH 8.6,0.5 mM MgCl₂. *Taq* and Pwo polymerase were tested using buffers according to Materials and Methods. Primer and template sequences are illustrated on the right.
- **Fig. 9:**: Application of DNA polymerase from *P. islandicum* in PCR. Reactions were carried out in a buffer containing 15 mM Tris-HCl pH 8.6, 12.5 mM KCI, 2.5 mM (NH₄)₂SO₄,1.25 mM MgCl₂ and 20 µg/ml BSA. 1 U of DNA polymerase from *P. islandicum* was used to amplify 500 bp (lane 1), 1000 bp (lane 2) and 1500 bp (lane 3) of a λ DNA template. Control PCR was performed with 2.5 U of High Fidelity enzyme (lane 4). 20 µl of each PCR product were applied on an 1% agarose gel..The corresponding molecular weights of the marker (lane 5) are indicated in kilobasepairs (kb).

### References

Blanco, L., Bernad, A., Blasco, M. A., & Salas, M. (1991) *Gene 100*, 27-38.
Bradford, M. M. (1976) *Anal. Biochem. 72*, 248-254.
Braithwaite, D. K., & Ito, J. (1993) *Nucleic Acids Res. 21,* 787-802.
Cariello, N. F., Swenberg, J. A., & Skopek, T. R. (1991) *Nucleic Acids Res. 19,* 4193 -4198.
Datukishvili, N., Pokholok, D., Lottspeich, F., Prangishvili, D., & Rechinsky, V. (1996) *Gene 177,* 271-273.
Dennis, P. P. (1997) *Cell 89*, 1007-1010.
DiRuggiero, J., & Robb, F. T. (1995) in *Archaea-a laboratory manual. Thermophiles* (Robb, F. T., & Place, A. R., Eds.) pp 97-99, Cold Sprinh Harbor Laboratory Press.
Dong, Q., Copeland, W. C., & Wang, T. S. (1993) *J. Biol. Chem. 268*, 24163-24174.
Edgell, D. R., Klenk, H. P., & Doolittle, W. F. (1997) *J. Bacteriol. 179,* 2632-2640.
Erlich, H. A., Gelfand, D. H., & Saiki, R. K. ( 1988) *Nature 331,* 461-462.
Huber, B., Kristjansson, J. K., & Stetter, K. O. (1987) *Arch. Microbiol. 149*, 95-101.
Keohavong, P., & Thilly, W. G. (1989) *Proc. Natl. Acad. Sci. USA 86*, 9253-9257.
Korge, B. P., Gan, S. Q., McBride, O. W., Mischke, D., & Steinert, P. M. (1992) *Proc. Natl. Acad. Sci. USA 89*, 910-914.
Kornberg, A., & Baker, D. (1992) *DNA replication,* Second ed., Freeman and company, New York. Laemmli, U. K. (1970) *Nature 227,* 680-685.
Longley, M. J., Bennett. S. E.. & Mosbaugh, D. W. (1990) *Nucleic Acids Res. 18,* 7317-7322. Lundberg, K. S., Shoemaker, D. D., Adams, M. W. W., Short, J. M., Sorge, J. A., & Marthur, E. J. *(1991) Gene 108,* 1-6.
Maniatis, T., Fritsch, E. F., & Sambrook. J. (1982) *Molecular cloning: a laboratory manual,* Cold Spring Harbor Laboratory, Cold Spring Harbor, N.Y.
Mullis, K., Faloona, F., Saiki, R., Horn, G., & Erlich, H. A. (1986) *Cold Spring Harbor Symp. Quant. Biol. 51,* 263-273.
Niehaus, F., Frey, B., & Antranikian, G. (1997) *Gene 204*, 153-158.
Perler, F. B., Kumar, S., & Kong, H. (1996) *Adv. Prot.* Chem. *48*,377-435.
Pisani, F. M., Martino, C. D., & Rossi, M. (1992) *Nucleic Acids Res. 20*, 2711-2716.
Prangishvili, D., & Klenk, H. P. (1993) *Nucleic Acids Res. 21*, 2768.
Sanger, F., Nicklen, S., & Coulson, A. R. (1977) *Proc. Natl. Acad. Sci. USA 74,* 5463-5467.
Schleper, C., DeLong, E. F., Preston, C. M., Feldman, R. A., Wu, K.-Y., & Swanson, R. V. (1998) J. *Bacteriol. 180,* 5003-5009.
Schleper, C., Swanson, R. V., Mathur, E. J., & DeLong, E. F. (1997) *J. Bacteriol. 179*, 7803-7811.
Schmidt, T. G. M., & Skerra, A. *(1994) J. Chromatogr. A 676,* 337-345.
Skerra, A. (1994) *Gene 151,* 131-135.
Southworth, M. W., Kong, H., Kucera, R. B., Ware, J., Jannasch, H. W., & Perler, F. B. (1996) *Proc. Natl. Acad. Sci. USA 93,* 5281-5285.
Spanos, A., & Hübscher, U. (1983) *Meth. Enzymol. 91, 263-277.*
Stetter, K. O. (1996) *FEMS Microbiol. Rev. 18,* 149-158.
Suzuki, K., Craddock, B. P., Kano, T., & Steigbigel, R. T. (1993 ) *Anal. Biochem. 210,* 277-281. Uemori, T., Ishino, Y., Doi, H., & Kato, I. (1995) *J. Bacteriol. 177,* 2164-2177.
Voss, S., & Skerra, A. (1997) *Protein Eng. 10, 975-982.*
Wang, T. S. F., Wong, S. W., & Korn, D. (1989) *FASEB J.* 3,14-21.
Woese, C. R., & Fox, G. E. (1977) *Proc. Natl. Acad. Sci. USA 74*, 5088-5090.
Wong, D. M., Weinstock, P. H., & Wetmur, J. G. (1991) *Nucleic Acids Res. 19*,2251-2259.

## Claims

1. A DNA polymerase obtainable from *Pyrobaculum islandicum* whereas six conserved motifs indicative of family B DNA polymerases were contained in the *Pyrobaculum islandicum* DNA polymerase sequence and whereas this polymerase exhibits 3'-5'-exonuclease activity.

2. A DNA polymerase according to claim 1 whereas the molecular weight of this polymerase is approximately 90 kDa.

3. A DNA polymerase according to claims 1 or 2 whereas the polymerase exhibits most activity at pH 7.3.

4. A DNA polymerase according to one of the claim 1-3 whereas this polymerase exhibits a low sensitivity to ddGTP.

5. A recombinant DNA polymerase according to one of the claims 1-4 obtainable from E.coli BL21 transformed by a vector containing a gene which encodes the DNA polymerase.

6. DNA encoding a polymerase according to one of the claims 1-4.

7. Vector containing a gene of a polymerase according to one of the claims 1-4.

8. Host transformed by a vector according to claim 7.

9. Method for producing a recombinant DNA polymerase according to claim 5.

10. Use of a DNA polymerase according to one of the claims 1-5 for in vitro amplification of DNA fragments.

11. Use of a DNA polymerase according to one of the claims 1-5 for determination of DNA sequences.

12. A method for cloning a B DNA polymerase using primers with SEQ ID No. 1 and SEQ ID No. 2 or variants thereof, the latter are selected from the following group: primers wherein one to three triplets are omitted and one to five triplets are added, primers wherein one or more of the inosins shown in SEQ ID No. 1 or SEQ ID No. 2 are substituted by a naturally occurring base or primers wherein one or more naturally occurring base are substituted by inosin in addition to the substitutions according to SEQ ID no. 1 or SEQ ID no. 2 wherein a naturally occurring base is substituted by inosin which is at the third position of the triplett.

13. A method of the amplification of fragments of B DNA polymerae using primers with SEQ ID No. 1 and SEQ ID No. 2 or variants thereof, the latter are selected from the following group: primers wherein one to three triplets are omitted and one to five triplets are added, primers wherein one or more of the inosins shown in SEQ ID No. 1 or SEQ ID No. 2 are substituted by a naturally occurring base or primers wherein one or more naturally occurring base are substituted by inosin in addition to the substitutions according to SEQ ID no. I or SEQ ID no. 2 wherein a naturally occurring base is substituted by inosin which is at the third position of the triplett.

## Patentansprüche

1. DNA-Polymerase, die aus Pyrobaculum islandicum erhältlich ist, worin sechs konservierte Motive, die für die Polymerasen der Familie B indikativ sind, in der Pyrobaculum islandicum DNA-Polymerasesequenz enthalten waren und wobei diese Polymerase 3'-5'-Exonucleaseaktivität zeigt.

2. DNA-Polymerase nach Anspruch 1, worin das Molekulargewicht dieser Polymerase etwa 90 kDa beträgt.

3. DNA-Polymerase nach Anspruch 1 oder 2, worin die Polymerase die meiste Aktivität bei einem pH von 7,3 zeigt.

4. DNA-Polymerase nach einem der Ansprüche 1 - 3, wobei diese Polymerase eine schwache Empfindlichkeit gegenüber ddGTP zeigt.

5. Rekombinante DNA-Polymerase nach einem der Ansprüche 1 - 4, die aus E.coli BL21, der mit einem Vektor, der ein Gen, das für die DNA-Polymerase kodiert, enthält, transformiert ist, erhältlich ist.

6. DNA, die eine Polymerase nach einem der Ansprüche 1 - 4 kodiert.

7. Vektor, der ein Gen einer Polymerase nach einem der Ansprüche 1 - 4 enthält.

8. Wirt, der mit einem Vektor nach Anspruch 7 transformiert ist.

9. Verfahren zur Herstellung einer rekombinanten DNA-Polymerase nach Anspruch 5.

10. Verwendung einer DNA-Polymerase nach einem der Ansprüche 1 - 5 für die in vitro Amplifizierung von DNA-Fragmenten.

11. Verwendung einer DNA-Polymerase nach einem der Ansprüche 1 - 5 für die Bestimmung von DNA-Sequenzen.

12. Verfahren zur Klonierung einer B-DNA-Polymerase unter Verwendung von Primern mit SEQ ID Nr. 1 und SEQ ID Nr. 2 oder Varianten davon, die letzteren sind aus der folgenden Gruppe gewählt: Primer, worin ein bis drei Tripletts weggelassen sind und ein bis fünf Tripletts hinzugefügt sind, Primer, worin ein oder mehrere der in SEQ ID Nr. 1 oder SEQ ID Nr. 2 gezeigten Inosine durch eine natürlich vorkommende Base substituiert sind oder Primer, worin eine oder mehrere natürlich vorkommende Basen durch Inosin zusätzlich zu den Substitutionen nach SEQ ID Nr. 1 oder SEQ ID Nr. 2 substituiert sind, worin eine natürlich vorkommende Base durch Inosin, das sich an Position 3 des Tripletts befindet, substituiert wird.

13. Verfahren zur Amplifikation von Fragmenten der B-DNA-Polymerase unter Verwendung von Primern mit SEQ ID Nr. 1 und SEQ ID Nr. 2 oder Varianten davon, die letzteren sind aus der folgenden Gruppe gewählt: Primer, worin ein bis drei Tripletts weggelassen sind und eins bis fünf Tripletts hinzugefügt sind, Primer, worin ein oder mehrere der in SEQ ID Nr. 1 oder SEQ ID Nr. 2 gezeigten Inosine durch eine natürlich vorkommende Base substituiert sind oder Primer, worin eine oder mehrere natürlich vorkommende Basen durch Inosin zusätzlich zu den Substitutionen nach SEQ ID Nr. 1 oder SEQ ID Nr. 2 substituiert sind, worin eine natürlich vorkommende Base durch Inosin, das sich an Position 3 des Tripletts befindet, substituiert wird.

## Revendications

1. ADN polymérase pouvant être obtenue à partir de Pyrobaculum islandicum, attendu que six motifs conservés significatifs de la famille des ADN polymérases B étaient contenus dans la séquence d'ADN polymérase de Pyrobaculum islandicum, et attendu que cette polymérase montre une activité 3'-5'-exonucléase.

2. ADN polymérase selon la revendication 1, attendu que la masse moléculaire de cette polymérase est de 90 kDa environ.

3. ADN polymérase selon les revendications 1 ou 2, attendu que la polymérase fait preuve d'une activité maximale à pH 7,3.

4. ADN polymérase selon l'une des revendications 1 à 3, attendu que cette polymérase fait preuve d'une faible sensibilité au ddGTP.

5. ADN polymérase recombinante selon l'une d es revendications 1 à 4, pouvant être obtenue à partir d'E.coli BL21 transformé par un vecteur contenant un gène qui code pour l'ADN polymérase.

6. ADN codant pour une polymérase selon l'une des revendications 1 à 4.

7. Vecteur contenant un gène d'une polymérase selon l'une des revendications 1 à 4.

8. Hôte transformé par un vecteur selon la revendication 7.

9. Procédé destiné à produire une ADN polymérase recombinante selon la revendication 5.

10. Utilisation d'une ADN polymérase selon l'une des revendications 1 à 5 pour l'amplification in vitro de fragments d'ADN.

11. Utilisation d'une ADN polymérase selon l'une des revendications 1 à 5 pour la détermination de séquences d'ADN.

12. Procédé destiné à cloner une ADN polymérase B au moyen d'amorces ayant les séquences SEQ ID No. 1 et SEQ ID No. 2, ou de variants de celles-ci, ces derniers étant choisis parmi le groupe suivant : des amorces dans lesquelles de un à trois triplets sont omis et de un à cinq triplets sont ajoutés, des amorces dans lesquelles une ou plusieurs des inosines représentées dans SEQ ID No. 1 ou SEQ ID No. 2 sont substituées par une base d'origine naturelle, ou des amorces dans lesquelles une ou plusieurs bases d'origine naturelle sont substituées par de l'inosine en plus des substitutions selon SEQ ID No. 1 ou SEQ ID No. 2 dans lesquelles une base d'origine naturelle est substituée par une inosine qui se trouve en troisième position du triplet.

13. Procédé permettant l'amplification de fragments d'ADN polymérase B au moyen d'amorces ayant les séquences SEQ ID No. 1 et SEQ ID No. 2, ou des variants de celles-ci, ces derniers étant choisis parmi le groupe suivant : des amorces dans lesquelles de un à trois triplets sont omis et de un à cinq triplets sont ajoutés, des amorces dans lesquelles une ou plusieurs des inosines présentées dans SEQ ID No. 1 ou SEQ ID No. 2 sont substituées par une base d'origine naturelle, ou des amorces dans lesquelles une ou plusieurs bases d'origine naturelle sont substituées par de l'inosine en plus des substitutions selon SEQ ID No. 1 ou SEQ ID No. 2 dans lesquelles une base d'origine naturelle est substituée par de l'inosine qui se trouve en troisième position du triplet.
